# EUROPEAN PATENT APPLICATION

(11) **EP 3 649 940 A1**
(43) Date of publication of application: **13.05.2020**
(21) Application number: 17917162.4
(22) Date of filing: 04.07.2017
(51) Int. Cl.: A61B 5/11

(54) **INFORMATION PROCESSING DEVICE, INFORMATION PROCESSING PROGRAM, AND INFORMATION PROCESSING METHOD**

(71) Applicant: Fujitsu Limited, Kawasaki-shi, Kanagawa 211-8588 (JP)
(72) Inventor: OYA, Takuro, Kawasaki-shi Kanagawa 211-8588 (JP); YAGINUMA, Yoshinori, Kawasaki-shi Kanagawa 211-8588 (JP)
(74) Representative: Haseltine Lake Kempner LLP
(86) International application number: PCT/JP2017/024453
(87) International publication number: WO 2019/008657

(57) **Abstract**

There is provided an information processing apparatus including: a movement extraction unit that extracts user's movement information obtained by quantifying movement of a user; an estimation unit that estimates recovery transition information from movement information and movement evaluation information of a specific rehabilitation subject person corresponding to the user's movement information, with reference to a recording unit that records movement information of a plurality of rehabilitation subject persons obtained by quantifying movement of the plurality of rehabilitation subject persons in association with movement evaluation information obtained by evaluating the movement information, on the basis of movement information and movement evaluation information of a rehabilitation subject person classified into a body type indicated by body type information of the user; and a selection unit that selects movement information that is to be a target of the user, on the basis of the estimated recovery transition information.

## Description

### TECHNICAL FIELD

The present invention relates to an information processing apparatus, an information processing program, and an information processing method.

### BACKGROUND ART

There is known a target achievement evaluation apparatus for appropriate and reasonable improvement anticipation and setting a target value for each trainee of different physical condition, age, and sexual distinction (see, for example, Patent Document 1).

According to Patent Document 1, a patient group is generated in which a plurality of patients are classified by physical condition, age, sexual distinction, and disorder severity, and movement information of a user and the patient group is analyzed. At that time, rehabilitation target movement information of the user is to be an average value of movement information of a patient group that is same as the user.

### CITATION LIST

### PATENT DOCUMENT

Patent Document 1: Japanese Laid-open Patent Publication No. 2001-420
Patent Document 2: Japanese Laid-open Patent Publication No. 2001-218746

### SUMMARY OF INVENTION

### TECHNICAL PROBLEM

However, using the average value of movement information of the plurality of patients grouped by attributes such as physical condition, sexual distinction, age, and disorder severity for the target movement information of the user, as in Patent Document 1, is not suitable for patients with symptoms that deviate from the average value, that is, symptoms whose average value is not the target movement, which causes a problem that the rehabilitation effect deteriorates. Furthermore, in Patent Document 1, since classification items to be grouped are fine and it is difficult to collect the number of samples suitable for users, reliability of the average value is low.

Therefore, in one aspect, an object of the present invention is to provide appropriate rehabilitation information to a user.

### SOLUTION TO PROBLEM

In one embodiment, there is provided an information processing apparatus including: a movement extraction unit that extracts user's movement information obtained by quantifying movement of a user; an estimation unit that estimates recovery transition information from movement information and movement evaluation information of a specific rehabilitation subject person corresponding to the user's movement information, with reference to a recording unit that records movement information of a plurality of rehabilitation subject persons obtained by quantifying movement of the plurality of rehabilitation subject persons in association with movement evaluation information obtained by evaluating the movement information, on the basis of movement information and movement evaluation information of a rehabilitation subject person classified into a body type indicated by body type information of the user; and a selection unit that selects movement information that is to be a target of the user, on the basis of the estimated recovery transition information.

### ADVANTAGEOUS EFFECTS OF INVENTION

In one aspect, the present invention can provide appropriate rehabilitation information to a user.

### BRIEF DESCRIPTION OF DRAWINGS

FIG. 1 is a diagram that illustrates an example of a hardware configuration of an information processing system according to an embodiment.
FIG. 2 is a diagram that illustrates an example of a hardware configuration of an information processing system according to an embodiment.
FIG. 3 is a diagram that illustrates an example of a functional configuration of a PC 4 according to an embodiment.
FIG. 4 is a flowchart that illustrates an example of patient's movement information collection processing according to an embodiment.
FIG. 5 is a view that illustrates an example of a body type model table, a sensor management table, and a rehabilitation time management table according to an embodiment.
FIG. 6 is a view that illustrates an example of a sensor information table according to an embodiment.
FIG. 7 is a view that illustrates an example of a movement management table according to an embodiment.
FIG. 8 is a view that illustrates an example of a patient-group movement database (DB) according to an embodiment.
FIG. 9 is a view for explaining classification and recovery transition of a body type group according to an embodiment.
FIG. 10 is a flowchart that illustrates an example of user's body type model generation processing according to an embodiment.
FIG. 11 (11A and 11B) is a flowchart that illustrates an example of user's recovery transition information selection processing according to an embodiment.
FIG. 12 is a view for explaining data in each table at a time of the recovery transition information selection processing according to an embodiment.
FIG. 13 is a view for explaining data in each table at a time of the recovery transition information selection processing according to an embodiment.
FIG. 14 is a view for explaining the user's recovery transition information selection processing according to an embodiment.
FIG. 15 is a view for explaining presentation of target movement to a user according to an embodiment.
FIG. 16 is a flowchart that illustrates an example of setting processing of a comparison period and an update period of movement according to an embodiment.
FIG. 17 is a view for explaining the comparison period and the update period of movement according to an embodiment.

### DESCRIPTION OF EMBODIMENTS

Hereinafter, an embodiment of the present invention is described with reference to the attached drawings. Note that, in this specification and the drawings, components having substantially the same functional configuration are denoted by the same reference numerals, and redundant explanations are omitted.

### [Hardware configuration of information processing system]

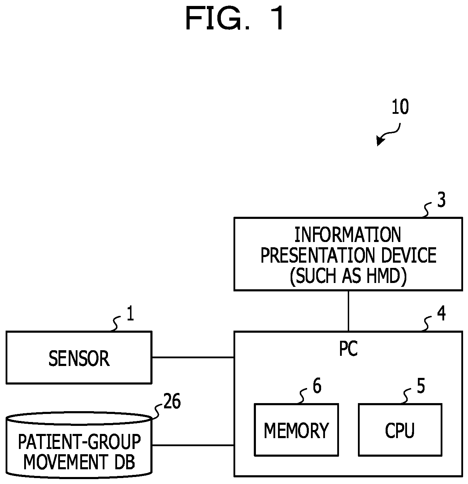

First, an example of a hardware configuration of an information processing system 10 according to an embodiment of the present invention is described with reference to FIG. 1. The information processing system 10 according to the present embodiment includes a personal computer (PC) 4 and an information presentation device 3.

The PC 4 is an example of an information processing apparatus, and can be applied to a tablet terminal, a smartphone, a personal digital assistants (PDA), and a wearable device. The PC 4 has a central processing unit (CPU) 5 and a memory 6. The CPU 5 acquires sensor data such as an acceleration or a speed from a sensor 1 such as an acceleration sensor or a gyro sensor, and quantifies movement of each patient on the basis of the acquired sensor data (for example, a step, a walking speed, and the like) to record in a patient-group movement DB 26.

The memory 6 has a random access memory (RAM), a read only memory (ROM), or a hard disk drive (HDD) for example as a component, and stores various types of tables and various types of programs. The memory 6 stores a program and data, such as a basic program and network settings. The CPU 5 uses an information processing program, other programs, and data recorded in the memory 6, to perform information processing such as patient's movement information collection processing and user's recovery transition information selection processing. Therefore, the CPU 5 realizes control of the entire apparatus and mounted functions. The PC 4 presents the information presentation device 3 with movement that is to be a user's target of rehabilitation. The information presentation device 3 may be wearable equipment such as a head mounted display (HMD) or face mounted display (FMD) worn by a user, a smartphone, a tablet terminal, a PC, or the like. In the present embodiment, the information presentation device 3 is described using an HMD worn by a user as an example.

In the information processing system 10 according to the present embodiment having such a configuration, for example, it is possible to provide a rehabilitation support service for stroke patients who cannot move normally due to paralysis, or a rehabilitation support service for assisting in restoring movement to a level of a healthy person.

Note that, for example, the sensor 1 is attached to a patient or a user, and temporally continuous sensor data is transferred from the sensor 1 to the PC 4. The sensor data acquired from the sensor 1 is used to estimate movement of the patient and the user to whom the sensor 1 is attached. The user is a person who receives provision of appropriate target rehabilitation information using this system. The patient is a rehabilitation subject person for whom rehabilitation movement information is collected in advance. The user is a person who receives rehabilitation information from this system, and can also be a rehabilitation subject person for whom rehabilitation movement information is collected.

### [Other hardware configuration of information processing system]

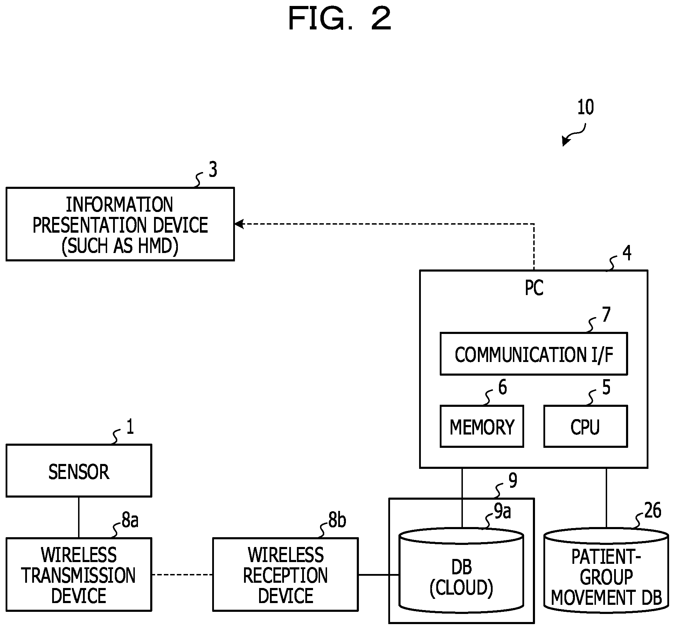

FIG. 2 illustrates an example of another hardware configuration of the information processing system 10 according to the present embodiment. In the information processing system 10 of FIG. 2, the PC 4 and a server 9 are connected to each other via a network. The sensor 1 worn by the user and the server 9 are connected via a wireless transmission device 8a and a wireless reception device 8b. The patient-group movement DB 26 may be recorded in a recording device of the PC 4, may be recorded in a recording device of the server 9, or may be recorded in a recording device on a cloud.

The server 9 has a database (DB) 9a that accumulates movement information of the user and the patient obtained by quantifying sensor data such as an acceleration detected by the sensor 1. The DB 9a may be recorded in a recording device in the server 9, or may be recorded in a recording device on a cloud. Note that the server 9 may be an arithmetic device on a cloud.

The PC 4 has a communication I/F 7 in addition to the CPU 5 and the memory 6. The communication I/F 7 is an interface to connect the PC 4 to the network. Therefore, the PC 4 performs data communication with other equipment such as the server 9 or the information presentation device 3, via the communication I/F 7. The PC 4 presents the information presentation device 3 of the HMD worn by the user with movement information that is to be a target of rehabilitation, via the communication I/F 7.

In the information processing system 10 of FIG. 2, the PC 4 and the server 9 are examples of the information processing apparatus. The server 9 may execute information processing such as the patient's movement information collection processing and the user's recovery transition information selection processing by using a program and data recorded in the memory 6, or the PC 4 may execute the information processing described above.

### [Functional configuration of PC]

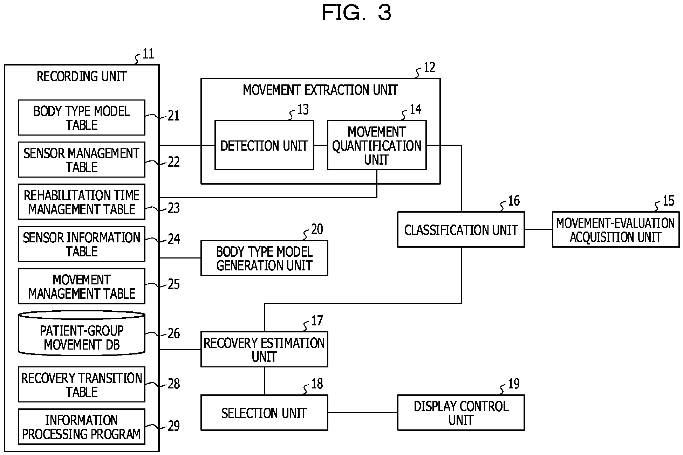

Next, an example of a functional configuration of the PC 4, which is an example of the information processing apparatus according to an embodiment, is described with reference to FIG. 3. The PC 4 includes a recording unit 11, a movement extraction unit 12, a movement-evaluation acquisition unit 15, a classification unit 16, a recovery estimation unit 17, a selection unit 18, a display control unit 19, and a body type model generation unit 20.

The recording unit 11 records a body type model table 21, a sensor management table 22, a rehabilitation time management table 23, a sensor information table 24, a movement management table 25, the patient-group movement DB 26, and a recovery transition table 28. Furthermore, the recording unit 11 records an information processing program 29.

The body type model table 21 records each of: a body type model generated from body type information of a user of the information processing system 10; and a body type model generated from body type information of a plurality of patients. The sensor management table 22 records an attachment position of the sensor 1 on the user or the patient. The rehabilitation time management table 23 records each of elapsed days from a rehabilitation start of the user and elapsed days from a rehabilitation start of the plurality of patients. The rehabilitation time management table 23 may record an elapsed time period or elapsed date and time from the rehabilitation start of the user and the patients. The sensor information table 24 records sensor data acquired from the sensor 1.

The movement management table 25 records movement information for each user and patient in which a sensing result of the sensor 1 is quantified into meaning to be understood by humans. When recording the movement information for each patient, the movement management table 25 records movement evaluation information obtained by evaluating the movement information by a medical staff, in association with the movement information. The medical staff may be present at a site where the patient performs rehabilitation, and the movement evaluation through visual inspection by the medical staff may be recorded in the movement management table 25 in association with the movement information for each of the rehabilitation elapsed days.

Examples of the quantified movement information include: movement of the whole body; a step; a walking speed; and an angle of each joint when a body joint is bent, twisted, or the like. When a medical staff such as a physical therapist or a doctor evaluates movement during rehabilitation, movement information for each patient is quantified such that the medical staff can understand and evaluate. Information obtained by quantifying the sensing data obtained from the sensor 1 attached to the user is also referred to as "user's movement information", and information obtained by quantifying the sensing data obtained from the sensor 1 attached to the patient is also referred to as "patient's movement information".

The patient-group movement DB 26 records: movement information of each patient obtained by quantifying sensing data of a plurality of patients; and movement evaluation information obtained by the medical staff evaluating the movement. The recovery transition table 28 records user's recovery transition information.

The movement extraction unit 12 includes a detection unit 13 and a movement quantification unit 14. The detection unit 13 detects sensor data such as an acceleration acquired from the sensor 1.

The movement quantification unit 14 quantifies the sensor data detected by the detection unit 13. Examples of quantified movement information include a walking speed and a step.

The movement-evaluation acquisition unit 15 acquires movement evaluation information obtained by a medical staff evaluating patient's movement. The classification unit 16 classifies (groups) quantified movement information of a plurality of patients into a plurality of body type groups.

The recovery estimation unit 17 is an example of an estimation unit that estimates user's recovery transition on the basis of specific patient's movement information approximate to user's movement information, movement evaluation information, and elapsed days from a rehabilitation start from movement information of patients in a body type group indicated by user's body type information, in the movement information of a plurality of patients in the classified body type group.

The recovery transition information indicates recovery transition estimated from movement information and movement evaluation information of a specific rehabilitation subject person corresponding to the user's movement information, on the basis of movement information and movement evaluation of a patient in a same body type group as the user's body type in classifying movement information of a plurality of patients for each body type. For example, the recovery transition may be a recovery inclination (speed) calculated from doctor's movement evaluation information or movement information for several days of the patient and an elapsed days from a rehabilitation start.

The selection unit 18 selects movement information that is to be a target of the user, on the basis of the estimated recovery transition information. The display control unit 19 causes the information presentation device 3 to display the selected movement information that is to be the target of the user. The body type model generation unit 20 generates a body type model of the user on the basis of the body type information of the user. The display control unit 19 may visualize the selected movement information to be the target into rehabilitation movement of the user's body type model, and may display on the information presentation device 3 by superimposing on a current movement of the user's body type model visualized from the user's movement information.

Functions of the movement extraction unit 12, the movement-evaluation acquisition unit 15, the classification unit 16, the recovery estimation unit 17, the selection unit 18, the display control unit 19, and the body type model generation unit 20 are realized, for example, by processing executed by the CPU 5 through the information processing program 29 installed in the memory 6. A function of the recording unit 11 is realized by the memory 6 for example.

### [Pre-processing: Patient's movement information collection processing]

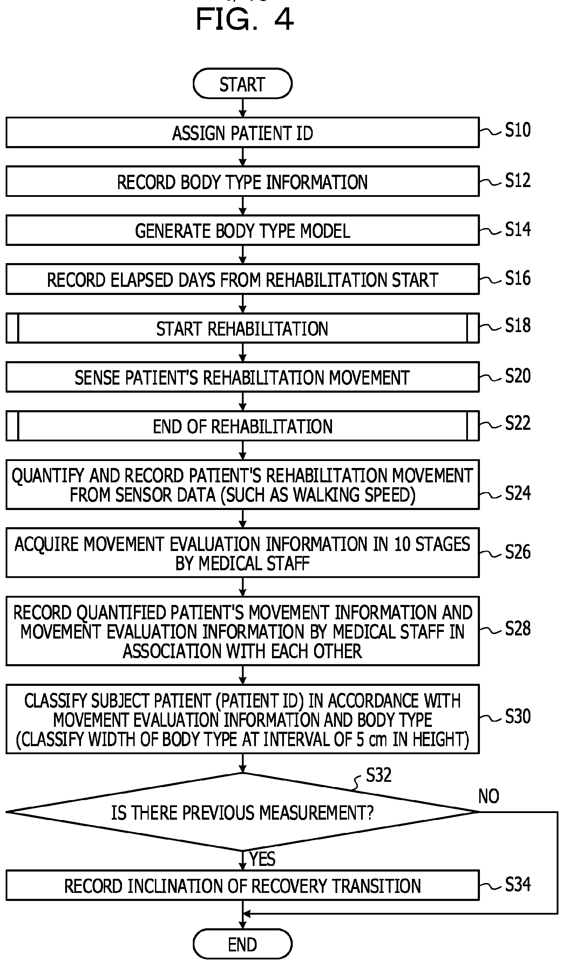

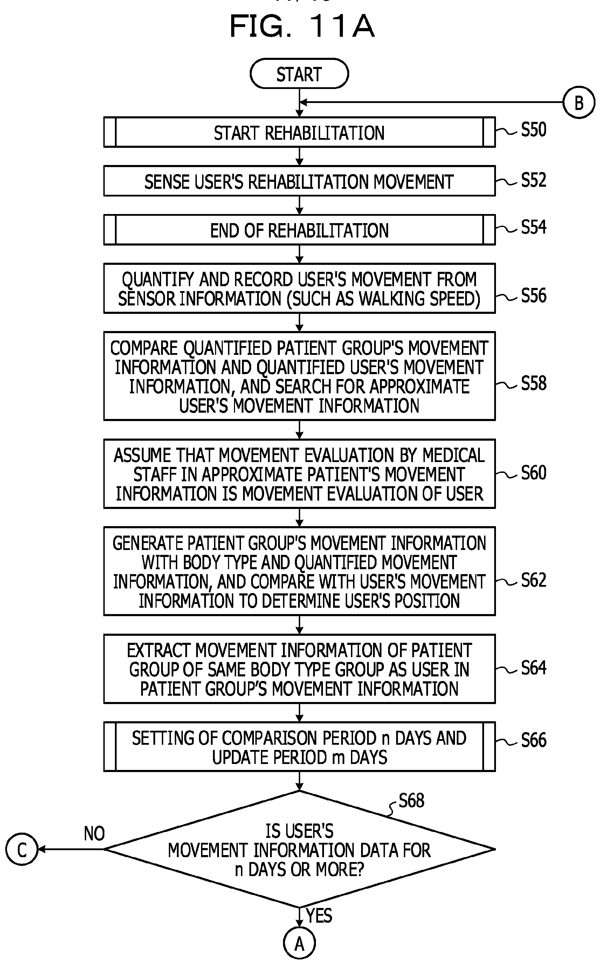

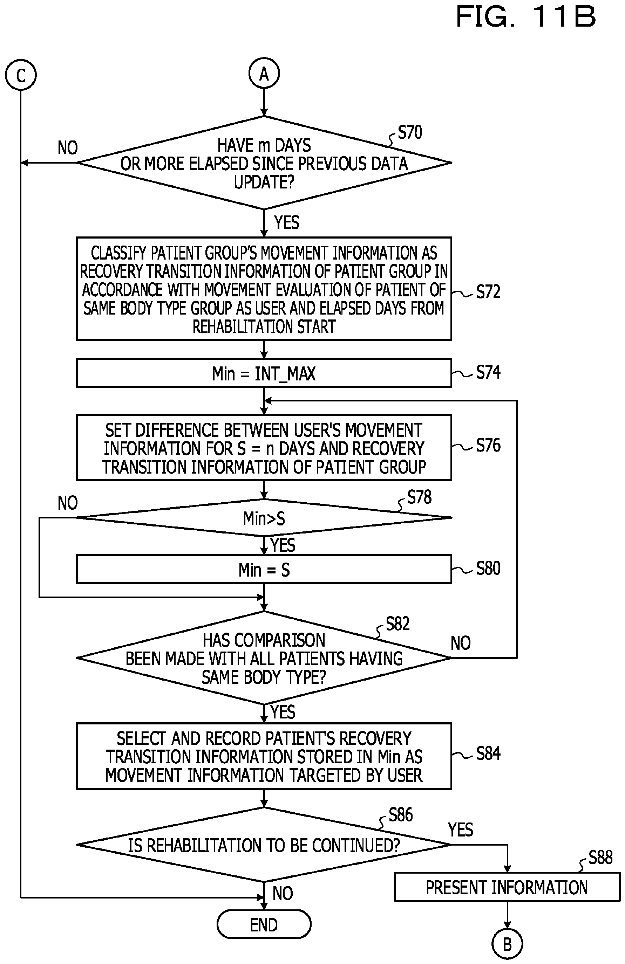

Next, an example of the patient's movement information collection processing according to the present embodiment is described with reference to FIG. 4. FIG. 4 is a flowchart that illustrates an example of processing for quantifying patient's movement and collecting patient's movement information and movement evaluation information, according to the present embodiment. The patient's movement, which is an example of the following movement of a rehabilitation subject person, is the patient's rehabilitation movement and daily movement. This stage is pre-processing of information collection performed before execution of the user's recovery transition information selection processing illustrated in FIG. 11.

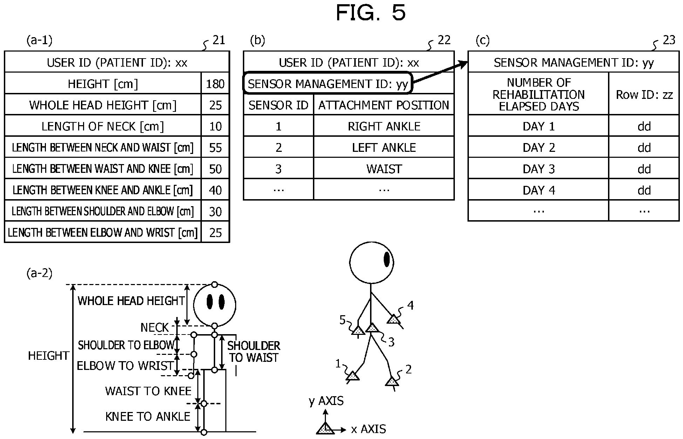

Note that the sensor management table 22 for managing a position of the sensor 1 attached to the patient has been generated before this processing is started. An example of the sensor management table 22 is illustrated in FIG. 5(b). For example, when five sensors with sensor IDs 1 to 5 are attached to the patient's right ankle, left ankle, waist, right wrist, and left wrist, information about the sensor ID and the attachment position is recorded in association with the sensor management ID.

As for the attachment position of the sensor 1, it is preferable to attach the sensor 1 on the whole body. This is because, by sensing movement of the whole body of the patient, all users can use the information processing system 10 according to the present embodiment even if movement varies depending on a type of illness.

Furthermore, the attachment position of the sensor 1 can be changed in accordance with a body site for which rehabilitation is performed. For example, it is preferable to attach the sensor 1 to the whole body and further attach the sensor 1 to a site for which the rehabilitation is performed. By sensing movement of the whole body and then sensing movement of the site for which the rehabilitation is performed, it is possible to evaluate the movement of both the whole body and the site of the rehabilitation target.

Moreover, the movement of the whole body may be sensed first, and thereafter, movement of only the rehabilitation target site may be sensed without sensing of movement of a part that is considered healthy. For example, in a case of foot rehabilitation, the sensor 1 may be attached to the whole body first, and then the sensor 1 may be attached to the foot and the waist. Therefore, it is possible to evaluate body part movement desired to be evaluated, and reduce a processing load.

When the processing of FIG. 4 is started, the detection unit 13 assigns a patient ID, which is identification information of the patient to which the sensor 1 is attached, for each patient (step S10). Next, the body type model generation unit 20 records the patient ID and body type information of the patient in the body type model table 21 (step S12).

FIG. 5(a-1) illustrates an example of the body type model table 21. In the body type model table 21 in FIG. 5(a-1), body type information such as a height of the patient is recorded in association with the patient ID.

Returning to FIG. 4, next, the body type model generation unit 20 generates a body type model of the patient from the body type information of the patient (step S14). Therefore, as illustrated in FIG. 5(a-2), a body type model reflecting a size of each patient such as height is generated. Note that the body type information illustrated in FIG. 5(a-1) and the body type model illustrated in FIG. 5(a-2) are examples, and the present invention is not limited to this.

Returning to FIG. 4, next, the detection unit 13 records elapsed days from the rehabilitation start in the rehabilitation time management table 23 (step S16). FIG. 5(c) illustrates an example of the rehabilitation time management table 23. In the rehabilitation time management table 23, a rehabilitation execution date (Row ID) is recorded in association with the number of rehabilitation elapsed days for each sensor management ID. The rehabilitation time management table 23 may manage an execution time of rehabilitation.

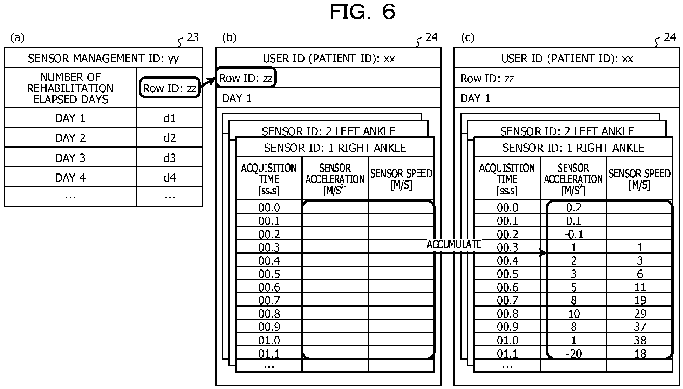

Returning to FIG. 4, next, when rehabilitation is started (step S18), the detection unit 13 senses patient's rehabilitation movement (step S20), and ends the rehabilitation (step S22). Specifically, the detection unit 13 detects sensor data from the sensor 1 attached to the patient and records in the sensor information table 24. FIGS. 6(b) and 6(c) illustrate an example of the sensor information table 24. The sensor information table 24 records a patient ID, a rehabilitation execution date, and sensor data (sensor acceleration, sensor speed) for each sensor identified by the sensor ID. FIG. 6(c) illustrates an example of a state in which sensor data is accumulated. In the present embodiment, only x-axis sensor data is recorded, but y-axis and z-axis sensor data may be recorded.

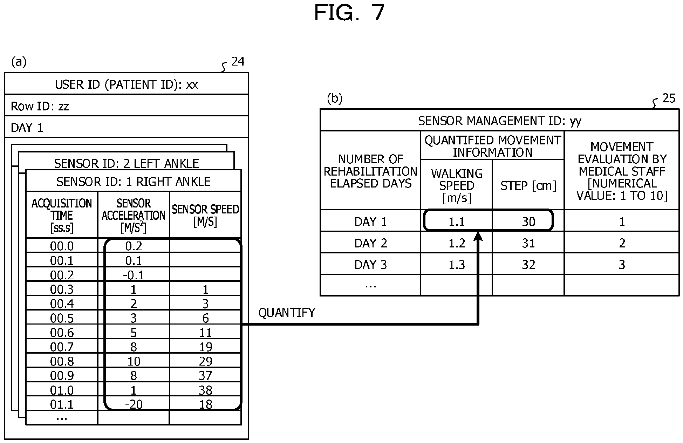

Returning to FIG. 4, next, the movement quantification unit 14 records patient's movement information obtained by quantifying patient's movement from the sensor data (step S24) . FIG. 7(b) illustrates an example of the movement management table 25 in which information obtained by quantifying patient's movement is recorded with use of sensor data of the acceleration and the speed of FIG. 7(a). In the present embodiment, a walking speed and a step are calculated as movement information obtained by quantifying patient's movement. However, these pieces of information are examples, and the information obtained by quantifying patient's movement is not limited to this, and may be, for example, an inclination of the body, a joint angle, or the like. For example, the movement quantification unit 14 calculates an average speed from the sensor on the waist as the walking speed. Furthermore, the movement quantification unit 14 estimates a moving distance from waveforms of the sensors on the both ankles, and calculates a step.

Returning to FIG. 4, next, the movement-evaluation acquisition unit 15 acquires the movement evaluation information in 10 stages by a medical staff (step S26), and the quantified patient's movement information and the movement evaluation information by the medical staff are associated with each other and recorded in the movement management table 25 (step S28). In the example of the movement management table 25 in FIG. 7(b), the movement evaluation information in 10 stages by the medical staff are assigned for each piece of the quantified movement information. However, performing the movement evaluation in 10 stages is an example, and the present invention is not limited to this.

Returning to FIG. 4, next, the classification unit 16 classifies a subject patient (patient ID) on the basis of movement evaluation information and a body type (step S30). For example, the classification unit 16 may classify a width of the body type at intervals of 5 cm in height. However, the classification method is not limited to this. For example, the classification may be performed with a height and a rehabilitation subject site (the arm in a case of arm fracture) as the classification subject.

Next, the recovery estimation unit 17 determines whether or not there is quantified patient's movement information measured previous time (step S32). When it is determined that there is no quantified patient's movement information measured previous time, the recovery estimation unit 17 ends this processing. When it is determined that there is quantified patient's movement information measured previous time, the recovery estimation unit 17 records an inclination of recovery transition in the recovery transition table 28 from the previous and current quantified patient's movement information (step S34), and ends this processing.

The processing illustrated in FIG. 4 is executed for each patient with respect to a plurality of patients, and quantified movement information of the plurality of patients is recorded in the patient-group movement DB 26.

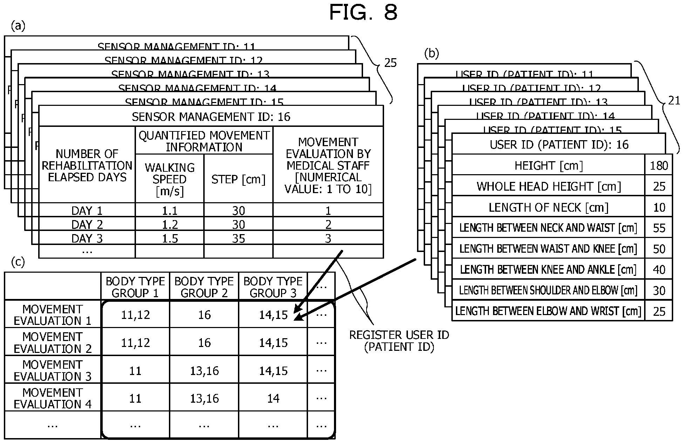

FIG. 8(c) illustrates an example of the patient-group movement DB 26 that records a result of classifying a subject patient (patient ID) on the basis of the movement evaluation information and the body type from the movement management table 25 of a plurality of patients (FIG. 8(a)) and the body type model table 21 of a plurality of patients (FIG. 8(b)). According to this, patients classified into a body type group 2 are the patient ID "16" for the movement evaluation 1, the patient ID "16" for the movement evaluation 2, the patient IDs "13" and "16" for the movement evaluation 3, and the patient IDs "13" and "16" for the movement evaluation 4.

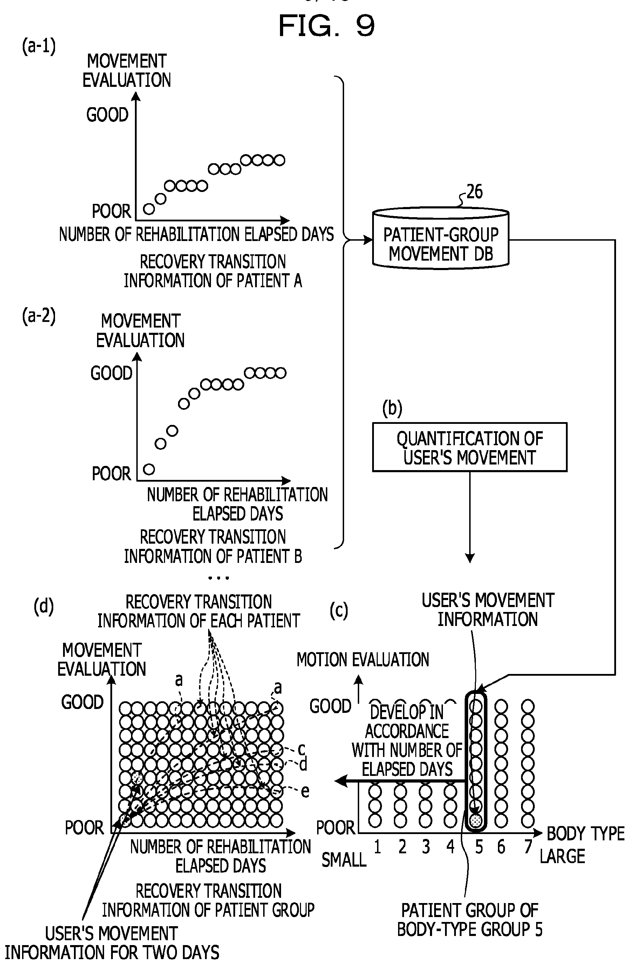

Furthermore, for example, FIG. 9 illustrates an example of body type group classification and an example of recovery transition. FIG. 9(a-1) and FIG. 9(a-2) illustrate movement information, movement evaluation information, and recovery transition information obtained from rehabilitation elapsed days of patients A and B. The recovery transition information (that is, the movement information, movement evaluation information, and rehabilitation elapsed days of patients A and B) is recorded in the patient-group movement DB 26. After rehabilitation information of recovery transition recorded in the patient-group movement DB 26 is classified by a same body type group as illustrated in FIG. 9(c), the recovery transition information for each patient of the same body type group 5 as the user is developed in accordance with the movement evaluation and the number of elapsed days from the rehabilitation start, as illustrated in FIG. 9(d). In FIG. 9 (c), the patient group is classified into a group of seven body type groups, but the present invention is not limited to this.

### [Body type model generation processing]

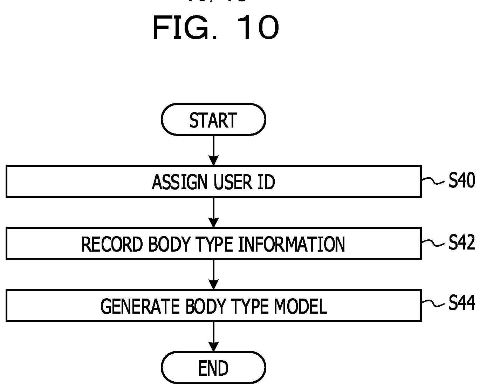

Next, an example of user's body type model generation processing according to the present embodiment is described with reference to FIG. 10. FIG. 10 is a flowchart that illustrates an example of the body type model generation processing according to an embodiment.

When this processing is started, the detection unit 13 assigns a user ID, which is identification information of the user to which the sensor 1 is attached (step S40). Next, the body type model generation unit 20 records the user ID and body type information of the user in the body type model table 21 (step S42).

The user's body type model table 21 records, in a pre-processing, body type information such as a height of the user in association with the user ID, similarly to the body type model table 21 of FIG. 5(a-1) in which the body type information of the patient is recorded.

Returning to FIG. 4, next, the body type model generation unit 20 generates a body type model of the user from the body type information of the user (step S44). Therefore, as illustrated in FIG. 5(a-2), a body type model reflecting a size of each user such as height is generated.

Note that, in the body type model table 21, size information related to the body type of each user and patient is recorded. For example, the body type model generation unit 20 may acquire size information related to the body type of each user and patient on the basis of image data or a motion capture, and record the size information in the body type model table 21.

The processing illustrated in FIG. 10 is executed for each user. Assuming that the user's body type does not change, the processing illustrated in FIG. 10 may be performed only for the first time when each user implements the information processing system 10. However, even for other than the first time, the user may regenerate his/her body type model when the body type changes.

### [Recovery transition information selection processing]

Next, an example of the user's recovery transition information selection processing according to the present embodiment is described with reference to FIG. 11. FIG. 11 is a flowchart that illustrates an example of the recovery transition information selection processing according to an embodiment. In this processing, user's movement is quantified, the quantified user's movement information and the patient group's movement information and movement evaluation information accumulated in the patient-group movement DB 26 are analyzed, recovery transition is estimated, and target movement is presented to the user.

Similarly to the pre-processing described above, before this processing is started, the sensor management table 22 for managing a position of the sensor 1 attached to the user has been generated (FIG. 5(b)). Then, the rehabilitation time management table 23 manages elapsed days (Row ID) from a rehabilitation start in association with the number of rehabilitation elapsed days for each sensor management ID (FIG. 5(c)). In the following embodiment, it is assumed that sensor data obtained by sensing user's movement for two days is acquired in advance in order to determine recovery transition.

When rehabilitation is started in this processing (step S50), the detection unit 13 senses user's rehabilitation movement (step S52) and ends the rehabilitation (step S54). Specifically, the detection unit 13 detects sensor data from the sensor 1 attached to the user and records in the sensor information table 24. FIGS. 6(b) and 6(c) illustrate an example of the sensor information table 24. The sensor information table 24 records a user ID, rehabilitation elapsed days, and sensor data for each sensor indicated by the sensor ID. FIG. 6(c) illustrates an example of a state in which sensor data is accumulated. In the present embodiment, only x-axis sensor data is described, but y-axis and z-axis sensor data may be additionally recorded.

Returning to FIG. 11, next, the movement quantification unit 14 performs movement recording on user's movement information such as a walking speed obtained by quantifying user's movement from the sensor information, in the movement management table 25 (step S56). Here, the movement evaluation information by a medical staff is unnecessary, thus is not recorded in the movement management table 25.

Next, the recovery estimation unit 17 compares the patient group's movement information recorded in the patient-group movement DB 26 with the quantified user's movement information, and the patient's movement information that is the same as or approximate to the user's movement is searched (step S58). Next, the recovery estimation unit 17 assumes that the movement evaluation by the medical staff in the same or approximate patient's movement information is the movement evaluation of the user (step S60).

Next, the recovery estimation unit 17 generates patient group's movement information with movement information quantified for each body type, and compares the user's movement information with the generated patient group's movement information to determine the user's movement evaluation position, that is, a current movement state (step S62). Next, the recovery estimation unit 17 extracts movement information of a patient group belonging to the same body type as the user in the patient group's movement information (step S64).

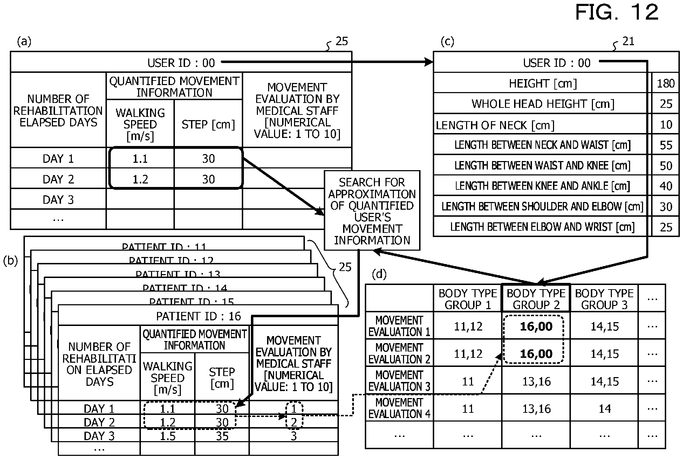

For example, as illustrated in FIGS. 12 (a) and 12(c), the user's movement management table 25 and the body type model table 21 are specified on the basis of the user ID. With use of these tables, patient's movement information that is same as or approximate to the user's movement information is extracted from the plurality of pieces of quantified patient's movement information in FIG. 12(b).

The extracted patient's movement information is classified for each body type. FIG. 12 (d) illustrates an example of patient IDs and user IDs classified for each body type and movement evaluation. Therefore, for example, in FIG. 12(d), it can be seen that the user's movement information with the user ID "00" corresponds to the movement evaluations 1 and 2 for the movement information of the patient ID "16" of the body type group 2 to which the user belongs. Therefore, as illustrated in FIG. 12(d), positions of the user are determined in the movement evaluation 1 column of the body type group 2 and the movement evaluation 2 column of the body type group 2. As described above, a user and a plurality of patients can be mapped by the movement evaluation and the body type.

For example, for the recovery estimation unit 17, for example, a description will be given to a case in which the body type group 5 that is the same as or approximate to the user is selected with use of the user's body type model table 21, among the patient groups grouped into seven body types illustrated in FIG. 9 (c) . In this case, as illustrated in FIG. 9(d), the number of elapsed days from a rehabilitation start and movement evaluation of the patient group's movement information of the selected body type group 5 are extracted, and the movement information for two days of the user acquired in advance is mapped. Therefore, a current position of the user with respect to recovery transition for each patient is determined. FIG. 9(d) indicates a current position of the user with a circle pointed by "user's movement information for two days".

Returning to FIG. 11, next, the recovery estimation unit 17 sets a comparison period n days and an update period m days (step S66). The comparison period n days only needs to be an integer equal to or greater than 1, and similarly, the update period m days only needs to be an integer equal to or greater than 1. For the comparison period n days, a minimum period (for example, the number of days) necessary for comparing movement of the user and the patient is set. For the update period m days, a minimum period (for example, the number of days) for updating the user's target movement is set.

Next, the recovery estimation unit 17 determines whether the user's movement information is data for n days or more (step S68). When it is determined that the user's movement information is not data for n days or more, the recovery estimation unit 17 ends this processing.

Whereas, when it is determined that the user's movement information is data for n days or more, the recovery estimation unit 17 determines whether m days or more have elapsed since the previous data update (step S70). When it is determined that m days or more have not elapsed since the previous data update, the recovery estimation unit 17 ends this processing.

Whereas, when it is determined that m days or more have elapsed since the previous data update, the recovery estimation unit 17 classifies the patient group's movement information as the recovery transition information of the patient group in accordance with the movement evaluation of the patient group of the same body type group as the user, and the elapsed days from the rehabilitation start (step S72) .

Next, the recovery estimation unit 17 inputs INT_MAX to a variable Min (step S74). To INT_MAX, a maximum value in the program is set. Next, the recovery estimation unit 17 sets a difference between user's movement information for n days (in the present embodiment, two days) and recovery transition information of the patient group, in a variable S (step S76). Next, the recovery estimation unit 17 determines whether the variable S is smaller than the variable Min (step S78). When the variable S is smaller than the variable Min, the recovery estimation unit 17 substitutes the value of the variable S into the variable Min (step S80), and proceeds to step S82. When the variable S is equal to or greater than the variable Min, the recovery estimation unit 17 immediately proceeds to step S82.

Next, the recovery estimation unit 17 determines whether the extracted body type has been compared with all patients (step S82). When it is determined that there is a patient that has not been compared among all patients having a same body type, the recovery estimation unit 17 returns to step S76, and performs processing of steps S76 to S80 for the patient that has not been compared. By comparing for the movement information of all the patients having the same body type in the processing of steps S76 to S80, a specific patient closest to the user's movement information can be determined.

In step S82, when the recovery estimation unit 17 determines that comparison has been made with all patients having the same body type, the selection unit 18 selects and records the patient's recovery transition information stored in the variable Min as the movement information targeted by the user (step S84).

Next, the display control unit 19 determines whether or not to continue rehabilitation (step S86). When it is determined that the rehabilitation is to be continued, the display control unit 19 presents the selected target movement (step S88), returns to step S50, and starts the rehabilitation again. Whereas, when it is determined that the rehabilitation is not to be continued, the display control unit 19 ends the processing.

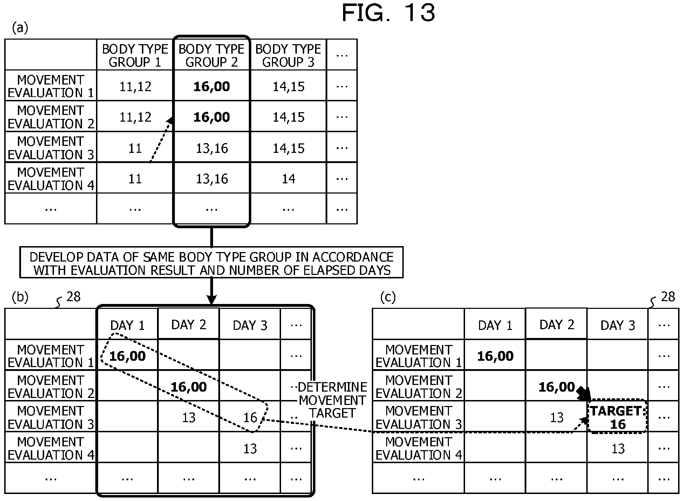

Therefore, according to FIG. 13(a) in which the user and a plurality of patients are mapped on the basis of the body type group and the movement evaluation, a patient with the patient ID "16" whose movement is approximate to that of a user with the user ID "00" is determined as the specific patient. FIG. 13(b) is an example of the recovery transition table 28 developed in accordance with the results of the movement evaluation and the number of elapsed days of the patient group of the body type group 2, and it can be seen that the patient with the patient ID "16" has obtained the evaluation of movement evaluation 1, 2, and 3 in accordance with the number of rehabilitation elapsed days. Therefore, as illustrated in FIG. 13(c), the movement of the movement evaluation 3 of the patient with the patient ID "16" that matches the determined position of the user with the user ID "00" is to be the user's target movement.

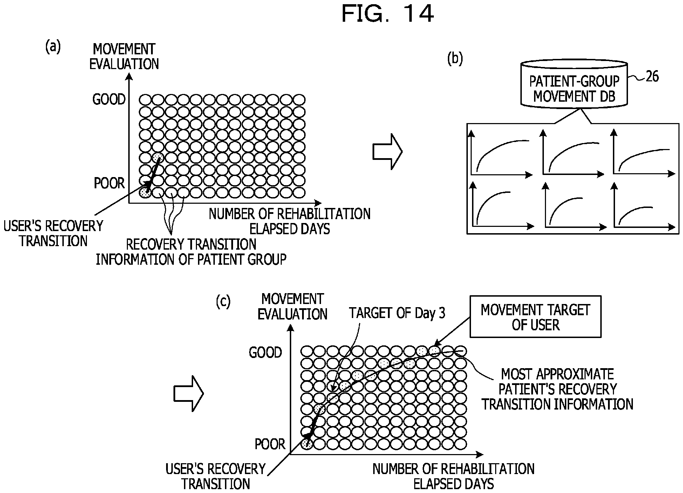

Therefore, it is assumed that the most approximate recovery transition information in FIG. 14(c) is selected for the user's recovery transition (result of rehabilitation movement for two days) in FIG. 14(a), among curves of recovery transition information of a plurality of patients recorded in the patient-group movement DB 26 in FIG. 14(b).

That is, as illustrated in FIG. 14(c), for the user's recovery transition for two days, the user's next target is specified to be the movement indicated by the movement information in which the number of elapsed days from the rehabilitation start indicated by the recovery transition information of the patient with the patient ID "16" is the third day.

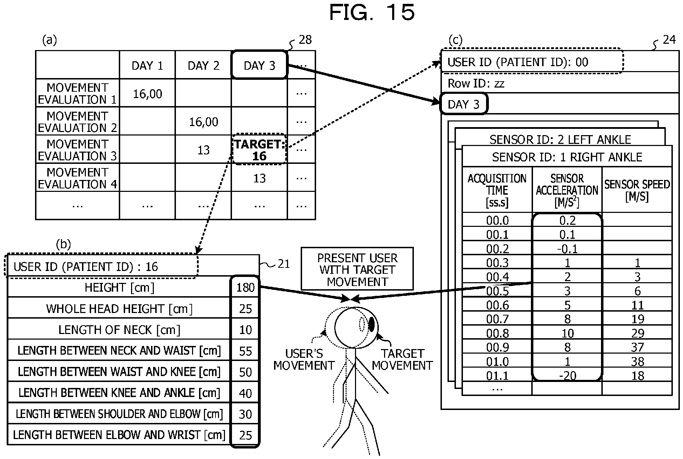

Therefore, the body type model of the patient with the patient ID "16" illustrated in FIG. 15(b) is extracted from the body type model table 21, patient's movement of the third day of the rehabilitation is reproduced from a sensor acceleration on the third day of the rehabilitation of the patient with the patient ID "16" illustrated in FIG. 15(c), and presented to the user as the target movement.

As described above, according to the information processing system 10 according to an embodiment, in rehabilitation performed by the user, a movement target can be selected from the patient group's movement information collected in advance, and presented to the user. Therefore, it is possible to provide the user with rehabilitation support that can make the user aware of maintaining appropriate rehabilitation movement, reduce compensatory movement, and use muscles that should be trained originally. As a result, rehabilitation can be performed efficiently.

Furthermore, on the basis of the movement evaluation of the patient's movement information collected in advance, a movement target suitable for the user can be presented. This enables rehabilitation only by a user without requiring the presence of a medical staff such as a physical therapist, and can reduce work costs for support by a medical staff and the like.

As illustrated in an example in FIG. 15, the presentation of the movement target is preferably displayed on the screen of the information presentation device 3 by superimposing a two-dimensional or three-dimensional target movement moving image and a current movement moving image of the user.

For the method of presenting a movement form on the screen, the method described in the specification of application number PCT/JP2017/000046 filed on January 4, 2017 may be used, or other methods may be used.

For example, by superimposing and displaying, on the screen, a state of the user after hospitalization and movement (selected target movement) of a patient that is a patient having the same body type as the user and recovered slightly as compared with the user, the user can clearly understand a difference between the current movement and the target movement. Therefore, it is possible to support users to regain their original movement before illness without requiring for rehabilitation specialists, and it can be expected to be useful for device and motivation for rehabilitation of users themselves, and discussions on rehabilitation policies or the like with a medical staff.

Note that, in the present embodiment, the movement information on the third day from the rehabilitation start of the selected patient has been set as the user's target movement, on the basis of the user's movement information on the first day and the second day from the rehabilitation start illustrated in FIG. 9 (d) However, the method for selecting movement information to be the target is not limited to this. For example, the target patient's movement information may be selected in accordance with the user's movement information for the immediately previous day, of the target patient's movement information may be selected in accordance with the user's movement information for the previous n days (n ≥ 2) . The target patient's movement information may be selected in accordance with the user's movement information at a predetermined rehabilitation execution date.

### [Comparison period and update period]

Recovery transition varies from person to person. Therefore, it is preferable to evaluate recovery transition of the user again at a predetermined interval and set recovery transition of a specific patient again as a movement target.

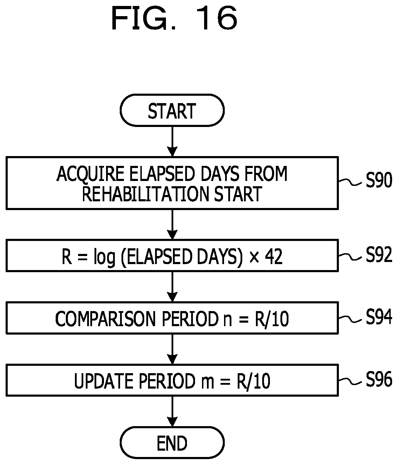

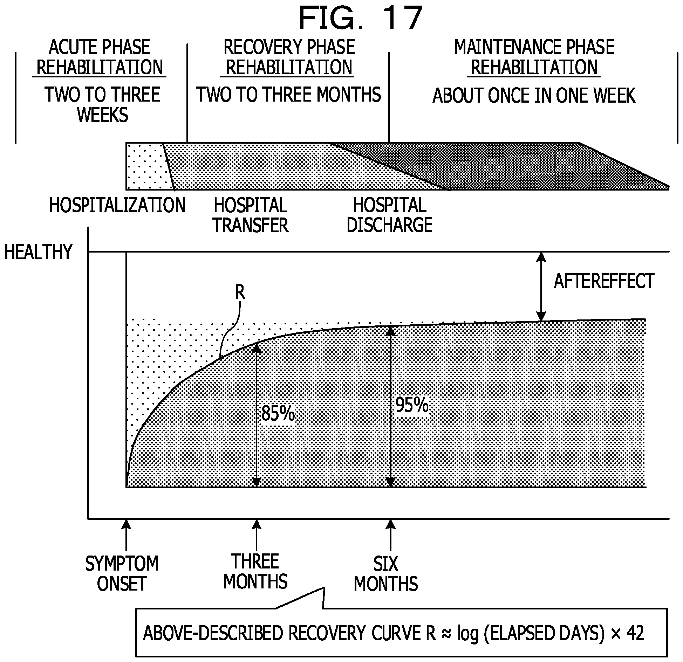

Here, a method for determining the update period m days and the comparison period n days for setting again as the movement target is described with reference to FIGS. 16 and 17. FIG. 16 is a flowchart that illustrates an example of setting processing of the comparison period and the update period of movement according to an embodiment. FIG. 17 is a view for explaining the comparison period and the update period of movement according to an embodiment.

As illustrated in FIG. 17, since recovery of movement by rehabilitation draws a parabola rather than a primary line, the recovery tends to be easy in an initial state and gradually becomes difficult. Therefore, the setting is made such that switching of the target movement is made early in an initial stage and switching of the target movement is made late in a later stage.

As an example, setting of the comparison period n and the update period m with use of a recovery curve formula as illustrated in FIG. 16 is described. The processing of FIG. 16 is started, and first, the recovery estimation unit 17 acquires elapsed days from a rehabilitation start (step S90). Next, the recovery estimation unit 17 substitutes the elapsed days into an equation R = log (elapsed days) x 42 of a recovery curve of movement by rehabilitation (step S92), and obtains the recovery curve R.

Next, the recovery estimation unit 17 substitutes a value of R/10 into the comparison period n (step S94), substitutes a value of R/10 into the update period m (step S96), and ends this processing. Therefore, an appropriate comparison period n and update period m can be calculated on the basis of the recovery curve R that changes depending on the elapsed days. This enables the comparison period n and the update period m to be set such that the switching of the target movement is made early in the initial stage of rehabilitation, and the switching of the target movement is made late in the later stage. Note that the equation of the recovery curve R according to the present embodiment is an example of a recovery curve, and the recovery curve may be determined by other method.

Although the information processing apparatus, the information processing program, and the information processing method have been described with reference to the embodiment described above, the information processing apparatus, the information processing program, and the information processing method according to the present invention are not limited to the embodiment described above, and various modifications and improvements can be made within the scope of the present invention. Furthermore, when there are a plurality of embodiments and modifications described above, they can be combined within a range that does not contradict each other.

For example, the configuration of the information processing system according to the embodiment described above is an example, and does not limit the scope of the present invention, and it is needless to say that there are various system configuration examples in accordance with the application and purpose. For example, the collection processing of movement information for a plurality of patients need not be performed by the PC 4 in advance, but may be executed by other equipment, and the movement information (recovery transition information) quantified for each patient may be recorded in the patient-group movement DB 26. A series of processing may be performed in cooperation with a plurality of computers in such a network system that a part of the processing performed by the PC 4 is executed by a server on a cloud and other processing is executed by the PC 4.

For example, in the embodiment, the rehabilitation movement of the patient is collected by the sensor as the rehabilitation subject person, but daily movement of the patient may be collected by the sensor 1 without being limited to the rehabilitation movement of the patient. Furthermore, without limiting to the patient, movement of an elderly person or movement of an athlete may be collected by the sensor 1. According to the present invention, for example, in a case of a gymnast, it is possible to present target movement for recovering to such an extent that gymnastics can be performed. For example, in a case of an elderly person, target movement that allows the elderly person to live alone can be presented.

### REFERENCE SIGNS LIST

- 1: Sensor
- 3: Information presentation device
- 4: PC
- 5: CPU
- 6: Memory
- 7: Communication I/F
- 8a: Wireless transmission device
- 8b: Wireless reception device
- 9: Server
- 10: Information processing system apparatus
- 11: Recording unit
- 12: Movement extraction unit
- 13: Detection unit
- 14: Movement quantification unit
- 15: Movement-evaluation acquisition unit
- 16: Classification unit
- 17: Recovery estimation unit
- 18: Selection unit
- 19: Display control unit
- 20: Body type model generation unit
- 21: Body type model table
- 22: Sensor management table
- 23: Rehabilitation time management table
- 24: Sensor information table
- 25: Movement management table
- 26: Patient-group movement DB
- 28: Recovery transition table
- 29: Information processing program

## Claims

1. An information processing apparatus comprising:
a movement extraction unit that extracts user's movement information obtained by quantifying movement of a user;
an estimation unit that estimates recovery transition information from movement information and movement evaluation information of a specific rehabilitation subject person corresponding to the user's movement information, with reference to a recording unit that records movement information of a plurality of rehabilitation subject persons obtained by quantifying movement of the plurality of rehabilitation subject persons in association with movement evaluation information obtained by evaluating the movement information, based on movement information and movement evaluation information of a rehabilitation subject person classified into a body type indicated by body type information of the user; and
a selection unit that selects movement information that is to be a target of the user, based on the estimated recovery transition information.

2. The information processing apparatus according to claim 1, further comprising
a display control unit that presents a user with the selected movement information to be the target.

3. The information processing apparatus according to claim 2, wherein
the display control unit visualizes the selected movement information to be the target into rehabilitation movement of a user's body type model, and displays by superimposing on movement of the body type model visualized from the user's movement information.

4. The information processing apparatus according to any one of claims 1 to 3, wherein
the estimation unit compares, for a specific comparison period for each specific update period, movement information of a rehabilitation subject person classified into a body type indicated by body type information of the user, with the user's movement information, and determines the specific rehabilitation subject person based on a comparison result.

5. The information processing apparatus according to claim 4, wherein
the estimation unit changes a specific update period and a specific comparison period in accordance with an elapsed time period from a start of movement.

6. An information processing program for causing a computer to execute processing of:
extracting user's movement information obtained by quantifying movement of a user;
estimating recovery transition information from movement information and movement evaluation information of a specific rehabilitation subject person corresponding to the user's movement information, with reference to a recording unit that records movement information of a plurality of rehabilitation subject persons obtained by quantifying movement of the plurality of rehabilitation subject persons in association with movement evaluation information obtained by evaluating the movement information, based on movement information and movement evaluation information of a rehabilitation subject person classified into a body type indicated by body type information of the user; and
selecting movement information that is to be a target of the user, based on the estimated recovery transition information.

7. The information processing program according to claim 6, wherein
the selected movement information to be the target is presented to a user.

8. The information processing program according to claim 7, wherein
the selected movement information to be the target is visualized into rehabilitation movement of a user's body type model, and displayed by being superimposed on movement of the body type model visualized from the user's movement information.

9. The information processing program according to any one of claims 6 to 8, wherein
for a specific comparison period for each specific update period, movement information of a rehabilitation subject person classified into a body type indicated by body type information of the user is compared with the user's movement information, and the specific rehabilitation subject person is determined based on a comparison result.

10. The information processing program according to claim 9, wherein
a specific update period and a specific comparison period are changed in accordance with an elapsed time period from a start of movement.

11. An information processing method in which a computer executes:
extracting user's movement information obtained by quantifying movement of a user;
estimating recovery transition information from movement information and movement evaluation information of a specific rehabilitation subject person corresponding to the user's movement information, with reference to a recording unit that records movement information of a plurality of rehabilitation subject persons obtained by quantifying movement of the plurality of rehabilitation subject persons in association with movement evaluation information obtained by evaluating the movement information, based on movement information and movement evaluation information of a rehabilitation subject person classified into a body type indicated by body type information of the user; and
selecting movement information that is to be a target of the user, based on the estimated recovery transition information.

12. The information processing method according to claim 11, wherein
the selected movement information to be the target is presented to a user.

13. The information processing method according to claim 12, wherein
the selected movement information to be the target is visualized into rehabilitation movement of a user's body type model, and displayed by being superimposed on movement of the body type model visualized from the user's movement information.

14. The information processing method according to any one of claims 11 to 13, wherein
for a specific comparison period for each specific update period, movement information of a rehabilitation subject person classified into a body type indicated by body type information of the user is compared with the user's movement information, and the specific rehabilitation subject person is determined based on a comparison result.

15. The information processing method according to claim 14, wherein
a specific update period and a specific comparison period are changed in accordance with an elapsed time period from a start of movement.
